Europäisches Patentamt

European Patent Office

Office européen des brevets

(19)

(11) Veröffentlichungsnummer: **0 350 435**
**A1**

(12) # EUROPÄISCHE PATENTANMELDUNG

(21) Anmeldenummer: **89730156.0**

(22) Anmeldetag: **03.07.89**

(51) Int. Cl.⁵: **A 61 F 2/30**
**A 61 L 27/00**

(30) Priorität: **04.07.88 DE 8808701**

(43) Veröffentlichungstag der Anmeldung:
**10.01.90 Patentblatt 90/02**

(84) Benannte Vertragsstaaten:
**AT BE CH DE ES FR GB GR IT LI NL SE**

(71) Anmelder: **MECRON MEDIZINISCHE PRODUKTE GMBH**
**Nunsdorfer Ring 23-29**
**D-1000 Berlin 48 (DE)**

(72) Erfinder: **Kranz, Curt, Dr.-Ing.**
**Kufsteiner Strasse 12**
**D-1000 Berlin 62 (DE)**

(74) Vertreter: **Christiansen, Henning, Dipl.-Ing.**
**Patentanwalt CHRISTIANSEN Pacelliallee 43/45**
**D-1000 Berlin 33 (DE)**

(54) **Endoprothese.**

(57) Prothese mit einer Oberflächenbeschichtung aus Hydroxilapatit, wobei eine makroskopische Struktur vorgesehen ist, die im wesentlichen ausschließlich Flächen aufweist, welche zur Ebene der die makroskopische Struktur aufweisenden Fläche um einen Winkel von vorzugsweise 45 bis 60° geneigt sind.

Fig. 1

Bundesdruckerei Berlin

EP 0 350 435 A1

# Beschreibung

## Endoprothese

Die Erfindung betrifft eine Prothese der im Oberbegriff des Anspruchs 1 angegebenen Art.

Bei derartigen Prothesen besteht das Problem, daß die Oberflächenbeschichtung bei starken Beanspruchungen zum Abrieb neigt. Das ist insbesondere dann nachteilig, wenn die Prothese beim Implantieren eingeschlagen werden muß oder in sonstiger Weise einer starken Oberflächenreibung ausgesetzt ist.

In diesem Fall gehen mit dem Abrieb der Hydroxilapatitschicht auch die mit dem Vorsehen dieser Schicht angestrebten Vorteile verloren.

Der Erfindung liegt die Aufgabe zugrunde, bei einer Prothese der eingangs genannten Gattung die Hydroxilapatitbeschichtung gegen Oberflächenbeanspruchungen bei der Implantation zu sichern.

Diese Aufgabe wird mit den kennzeichnenden Merkmalen des Anspruchs 1 gelöst.

Besonders vorteilhaft bei der Erfindung ist insbesondere, daß mit den erfindungsgemäßen Maßnahmen auch der Sitz der Prothese in der Weise verbessert wird, daß ein intensiverer Eingriff in die Knochenstruktur erfolgt.

Besonders vorteilhaft ist es, wenn die makroskopische Struktur bevorzugt pyramidenförmige Körper aufweist mit Abmessungen, die einer Grundfläche von 0,5 bis 1,5 mm² entsprechen, wobei die Pyramiden mit einer Hydroxil-Apatit (Hydroxyapatit $Ca_{10}(PO_4)_6(OH)_2$) Beschichtung von einer Dicke zwischen 30 und 100 μm versehen sind. Dieses Konzept der Krafteinleitung in die Prothese zeichnet sich insbesondere dadurch aus, daß die Mischbeanspruchungen, die durch die schrägen Flächen entstehen (Druck, Zug und ermöglicht die erfindungsgemäße Oberflächengestaltung ein optimales Anwachsen an die benachbarte Knochenoberfläche, da hier die örtlichen Kraftspitzen bei den auftretenden Belastungen relativ gering sind. Wegen der großen beteiligten Oberflächenbereiche der Prothese ist die Haltewirkung nach erfolgtem Einwachsen jedoch groß, so daß somit wirkungsvolle Maßnahmen gegen eine Prothesenauslockerung getroffen sind.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 eine vergrößerte Wiedergabe eines Ausführungsbeispiels einer Teiloberfläche der erfindungsgemäßen Prothese,

Figur 2 die Teildarstellung des Ausführungsbeispiels gemäß Figur 1 im Schnitt sowie

Figur 3 das Ausführungsbeispiel insgesamt in Seitenansicht.

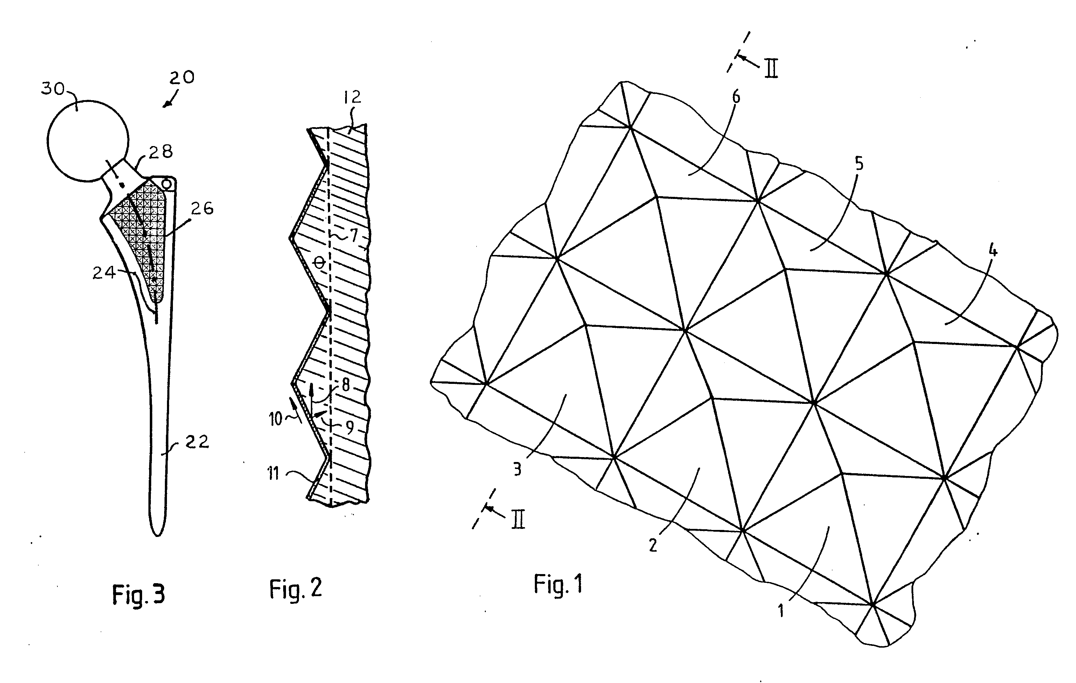

In Figur 1 ist als Auschnitt die makroskopische Pyramidenstruktur erkennbar, wobei vierseitige Pyramiden 1 bis 6 gleicher Größe die Oberfläche des Implantats 7 dicht bedecken. Die Pyramidenflächen bilden mit ihren Grundflächen, welche mit der Hauptoberflächenebene der Prothese zusammenfallen, einen Winkel von im wesentlichen 50°. Dieser kann entsprechend den Anforderungen variiert werden, Scherung), die reine Schäl- und Scherbeanspruchung der Verbindungsschicht zum Grundmaterial reduzieren. Auf diese Weise läßt sich die Lebensdauer der Verbindung zwischen dem Prothesengrundmaterial - bevorzugt Titan - und der Beschichtung erheblich verlängern, so daß sich das Implantationsverhalten verbessert, damit die Implantationszeit verlängert und Reoperationen vermieden werden.

Das An- bzw. Einwachsen wird insbesondere begünstigt, wenn die pyramiden- oder kegelstumpfförmigen Erhebungen in ihren Fußbereichen unmittelbar aneinandergrenzen, so daß die Mantelflächen in den Talbereichen in einem Winkel aneinandergrenzen, der der dem doppelten Winkel der Neigung der Mantelflächen zur Richtung der Gesamtoberfläche der Prothese entspricht. Auf diese Weise wird die Haftung zwischen Knochenmaterial und Prothesenoberfläche in der Weise begünstigt, als beide Materialien mit in etwa gleicher (spiegelbildlicher) Oberflächenform ineinandergreifen und die bei Belastung angreifenden Kräfte in relativ großen Flächenbereichen übergeleitet werden. Der "Abbau" der örtlichen Belastungen beider Materialien in Richtung auf das jeweils andere Material entspricht dabei in etwa dem jeweils zur Verfügung stehenden tragenden Querschnitt.

Die erfindungsgemäße Oberflächengestaltung wird in vorteilhafter Weise eingesetzt insbesondere bei Hüftgelenkprothesen wobei im dorsalen und ventralen proximalen Schaftbereich das Anwachsen durch die beschriebene Mikrostruktur unterstützt wird. Durch die Anbringung im ventralen und/oder dorsalen Bereich am proximalen Schaftende wobei ein vergrößerter Winkel die Festigkeit der Oberfläche Beschichtung aus Hydroxil-Apatit weist ein Dicke von ca. 50 μ auf.

Der Vorteil der Erfindung besteht im implantierten Zustand darin, daß die Krafteinleitung bei "Anwachsen" der Prothese durch Abbau von Hydroxil-Apatit wesentlich verbessert ist. Insbesondere bei Mischbeanspruchung, wird infolge der zur Grundfläche geneigten Flächen die Schäl-und Scherbeanspruchung der Verbindungsschicht zum Grundmaterial reduziert.

Bei der in Figur 2 dargestellten Schnittdarstellung ist ersichtlich, daß durch die pyramidenförmige Anordnung in Richtung der Prothesenhauptoberfläche 7 verlaufende Scherkräfte 8 in eine senkrecht zur Pyramidenoberfläche wirkende Kraftkomponente 9 und eine verringerte Scherkraftkomponente 10 zerlegt werden. Das Anhaften der Beschichtung bzw. des Körpergewebes nach der Resorption der Beschichtung wird noch unterstützt durch eine Porosität der Oberfläche. Die Beschichtung 11 ist in Figur 2 unmaßstäblich vergrößert dargestellt.

Eine mit der erfindungsgemäßen Beschichtung versehene Schaftprothese 20 mit einem Hals 28, einem Schaft 22 und einer Gelenkkugel 30 ist in Figur 3 wiedergegeben. Die erfindungsgemäße Struktur ist im dorsalen und ventralen proximalen Bereich 26 der Schaftprothese vorgesehen (entsprechend der Vor- bzw. Rückseite der Zeichnungsebene, die der Fläche entspricht, in dem der am stärksten gekrümmte Bereich der die Krümmung der Prothese bestimmende Linien 24 verläuft). Die Mikrostruktur ist dabei im Bereich der größten Schaftkrümmung in einem Oberflächenbereich vorgesehen, der im wesentlichen parallel zu einer (gedachten) Ebene verläuft, der die die Krümmung bestimmende, strichpunktiert dargestellte, Linie 24 enthält. Die letztgenannte Linie verläuft durch die Schwerpunkte der Schnittebenen, welche ihrerseits senkrecht zu dieser Linie gerichtet sind. Der genannte Oberflächenbereich erstreckt sich in jeweils einem etwa dreieckförmigen Bereich, der durch die schmalen Seitenflächen und die Abschlußfläche im gelenknahen Bereich des Prothesenschaftes begrenzt wird. Der Schaft im proximalen Bereich 26, der die Mikrostruktur trägt, eine im wesentlichen konstante Dicke (senkrecht zur Zeichenebene) auf, die der Breite des Schaftes am unteren Ende des Bereichs 26 entspricht, wobei die Kanten verrundet sind. Der daran anschließende Schaftbereich weist bis zum unteren Ende hin einen im wesentlichen runden Querschnitt auf.

Damit ergibt sich ein verhältnismäßig großflächiger Bereich des Schaftes in der Nähe des Gelenks, in dem durch die erfindungsgemäße Mikrostruktur mit Beschichtung das Anwachsen in Verbindung mit der erfindungsgemäßen Oberflächenstruktur ein guter zusätzlicher Halt des Schaftes hervorgerufen wird. Hierdurch gelingt es, Spitzenbelastungen, die in anderen Prothesenbereichen auftreten abzubauen und zu begrenzen, so daß ein Auslockern des Schaftes insgesamt verhindert ist.

Die Erfindung beschränkt sich in ihrer Ausführung nicht auf das vorstehend angegebene bevorzugte Ausführungsbei spiel. Vielmehr ist eine Anzahl von Varianten denkbar, welche von der dargestellten Lösung auch bei grundsätzlich anders gearteten Ausführungen Gebrauch machen.

## Patentansprüche

1. Prothese mit einer Oberflächenbeschichtung aus Hydroxilapatit, **dadurch gekennzeichnet,** daß ein Bereich mit einer makroskopischen Struktur vorgesehen ist, der ausschließlich Flächen aufweist, welche zur Ebene der die makroskopische Struktur aufweisenden Fläche um einen Winkel von im Bereich von 45 bis 60° geneigt sind.

2. Prothese nach Anspruch 1, **dadurch gekennzeichnet,** daß die Struktur aus kegel- oder pyramidenförmigen Körpern als Grundkörpern besteht.

3. Prothese nach Anspruch 2, **dadurch gekennzeichnet,** daß die kegel- oder pyramidenförmigen Körper eine Grundfläche von im wesentlichen 0,5 bis 1,5 mm² aufweisen.

4. Prothese nach einem der vorangehenden Ansprüche, **dadurch gekennzeichnet,** daß die Beschichtung eine Dicke von 30 bis 100 μm aufweist.

5. Prothese nach einem der vorangehenden Ansprüche, daß die geneigten Flächen in zwischen den Erhebungen gebildeten Talbereichen unmittelbar aneinandergrenzen.

6. Prothese nach einem der vorangehenden Ansprüche, **dadurch gekennzeichnet,** daß die Struktur im dorsalen und/oder ventralen Bereich (26) einer Schaftprothese (22) vorgesehen ist.

7. Prothese nach einem der vorangehenden Ansprüche, **dadurch gekennzeichnet,** daß die Struktur im proximalen Bereich (26) einer Schaftprothese (22) vorgesehen ist.

8. Prothese nach einem der vorangehenden Ansprüche, **dadurch gekennzeichnet,** daß die Struktur bei einer Schaftprothese (22) im Bereich der größten Schaftkrümmung in mindestens einem Oberflächenbereich (26) vorgesehen ist, der im wesentlichen parallel zur Ebene, in der die die Krümmung bestimmende Linie (24) verläuft, gerichtet ist.

Fig.1

Fig.2

Fig.3

## EINSCHLÄGIGE DOKUMENTE

EP 89730156.0

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| Y | <u>WO - A1 - 86/06 617</u><br>(PLASMAINVENT)<br>* Seite 3, Zeilen 11-21;<br>Seite 7, Zeilen 6-18; Fig.<br>1 *<br>-- | 1 | A 61 F   2/30<br>A 61 L 27/00 |
| Y | <u>EP - A1 - 0 311 749</u><br>(HOWMEDICA)<br>* Ansprüche 1,10 *<br>-- | 1 | |
| A | <u>DE - A1 - 3 717 818</u><br>(KOGAKU)<br>* Spalte 2, Zeilen 1-7 *<br>---- | 1 | |

RECHERCHIERTE
SACHGEBIETE (Int. Cl. 4)

A 61 F
A 61 L

Der vorliegende Recherchenbericht wurde für alle Patentansprüche erstellt.

| Recherchenort | Abschlußdatum der Recherche | Prüfer |
|---|---|---|
| WIEN | 09-10-1989 | MIHATSEK |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82